# EUROPÄISCHE PATENTSCHRIFT

(11) **EP 0 448 798 B1**
(45) Veröffentlichungstag und Bekanntmachung des Hinweises auf die Patenterteilung: **26.07.1995**
(21) Anmeldenummer: 90123741.2
(22) Anmeldetag: 10.12.1990
(51) Int. Cl.: A61B 17/39

(54) **HF-Chirurgiegerät**
Electrosurgical unit
Electro-bistouri

(30) Priorität: 27.03.1990 DE 4009819
(43) Veröffentlichungstag der Anmeldung: 02.10.1991
(73) Patentinhaber: SIEMENS AKTIENGESELLSCHAFT, 80333 München (DE)
(72) Erfinder: Altendorf, Hans-Walter, Dipl.-Ing., W-6520 Worms 25 (DE)

(56) Entgegenhaltungen:
- EP-A- 0 219 568
- EP-A- 0 316 469
- EP-A- 0 368 532
- GB-A- 2 164 473

## Beschreibung

Die Erfindung betrifft ein HF-Chirurgiegerät mit einem Mikroprozessor und mit einer Vergleichsschaltung, die ein Steuersignal abgibt, das auf eine gleichgerichtete Betriebsspannung einwirkt, die ein HF-Ausgangssignal steuert, das mit einem Modulationssignal modulierbar ist.

Aus der EP-A-0 316 469 ist ein HF-Chirurgiegerät der eingangs genannten Art bekannt. Bei dem bekannten HF-Chirurgiegerät können einer Vergleichsschaltung verschiedene Sollwerte für eine HF-Ausgangsspannung zugeführt werden. Die Sollwerte können vorprogrammiert und durch Betätigen von Schaltmitteln, z.B. an einem Elektrodenhandgriff, auch aus einem Mikroprozessor abrufbar sein. Das erforderliche Vergleichssignal wird aus der HF-Ausgangsspannung gebildet, wozu ein isolierendes Bauteil, z.B. ein Transformator, und mindestens ein Spannungswandler erforderlich sind. Daher muß das HF-Ausgangssignal eine möglichst konstante Struktur haben, was bei einer Amplitudenmodulation nicht der Fall ist. Bei dem bekannten HF-Chirurgiegerät ist zu prüfen, ob das Vergleichssignal dem Spitzenwert, dem Effektivwert oder einem Mittelwert der modulierten HF-Ausgangsspannung proportional sein soll. Dementsprechend sind zusätzliche Spannungswandler erforderlich, die im Bedarfsfall vom Bediener einzuschalten sind.

Um eine tiefe Koagulation zu erreichen, wird durch die Modulation das Verhältnis der Spitzenspannung zur Effektivspannung vergrößert, wobei jedoch die effektive Ausgangsleistung weitgehend konstant bleiben muß. Durch die Amplitudenmodulation entstehen in der HF-Ausgangsspannung entsprechend der zugehörigen Hüllkurve des Modulationssignals Leistungserhöhungen bzw. Leistungsverminderungen. Die HF-Ausgangsspannung hat daher keine konstante Struktur. Folglich entsteht bei einer z.B. vom Spitzenwert der HF-Ausgangsspannung abhängigen Regelung durch die Täler ein gravierender Rückgang der verfügbaren effektiven Ausgangsleistung. Sonach ist keine befriedigende Koagulation möglich.

Der Bediener des bekannten HF-Chirurgiegerätes ist daher gezwungen, durch Umschalten auf einen anderen Spannungswandler und eine höhere Ausgangsspannung den durch die Modulation auftretenden Leistungsabfall auszugleichen. Das ist aber insofern mit Schwierigkeiten verbunden, als zwischen der Betriebsspannung und der HF-Ausgangsleistung ein nichtlinearer Zusammenhang besteht. Vor allem der weniger geübte Bediener eines HF-Chirurgiegerätes kann mit dem Auswählen und Einstellen der geeigneten Parameter schnell überfordert sein.

Aufgabe der Erfindung ist es, ein HF-Chirurgiegerät der eingangs genannten Art anzugeben, mit dem bei geringem Bauteileaufwand im Falle einer Umschaltung auf ein moduliertes HF-Ausgangssignal eine selbsttätige, weitgehende Konstanz der effektiven Ausgangsleistung erreicht wird. Ferner sollen an das modulierte HF-Ausgangssignal Zusatzeinrichtungen, z.B. Einrichtungen zur Überwachung des Ausgangssignals, ebenfalls selbsttätig angepaßt werden.

Diese Aufgabe wird erfindungsgemäß durch die im Anspruch angegebenen Merkmale gelöst. In den übrigen Ansprüchen sind vorteilhafte Ausgestaltungen und Weiterbildungen der Erfindung angegeben.

Bei der erfindungsgemäßen Ausbildung sind weder ein isolierender Transformator noch Spannungswandler zur Erzeugung eines Vergleichssignals erforderlich. Da der gemäß der Erfindung im Mikroprozessor (Kleinstrechner) gebildete Sollwert aus einem Stellsignal für die vorgewählte HF-Ausgangsleistung in Abhängigkeit von einem Modulationssignal errechnet wird, entfällt für den Bediener bei Umschaltung auf ein moduliertes HF-Ausgangssignal jegliche manuelle Anpassungstätigkeit hinsichtlich der effektiven HF-Ausgangsleistung. Der letztgenannte wesentliche Vorteil der Erfindung tritt auch bei einer Änderung des Modulationssignales, z.B. zur Veränderung der Koagulationstiefe, ein. Die Struktur des Modulationssignals ist bekannt bzw. im Mikroprozessor bestimmbar. Daher kann der Prozessor einen Sollwert erzeugen, der alle sich durch die Modulation ändernden Parameter im voraus berücksichtigt.

In Ausbildung der Erfindung können mit Hilfe des Mikroprozessors aus dem Modulationssignal, das zur Einsparung eines entsprechenden Generators auch im Mikroprozessor erzeugt werden kann, weitere Stell-, Korrektur-und/oder Hilfssignale gebildet werden, die insbesondere im Falle eines modulierten HF-Ausgangssignales zur Steigerung der Genauigkeit und/oder Leistungsfähigkeit auf Zusatzeinrichtungen, wie z.B. Überwachungs-, Anzeige- und/oder Instrumentenbeleuchtungseinrichtungen, einwirken.

Weitere Vorteile und Einzelheiten der Erfindung ergeben sich aus der nachfolgenden Beschreibung eines Ausführungsbeispiels anhand der Zeichnungen und in Verbindung mit den Ansprüchen.

Es zeigen:
Figur 1 ein Prinzipschaltbild eines erfindungsgemäßen HF-Chirurgiegerätes und
Figuren 2 und 3 Hüllkurven von unterschiedlich modulierten HF-Ausgangssignalen des erfindungsgemäßen HF-Chirurgiegerätes.

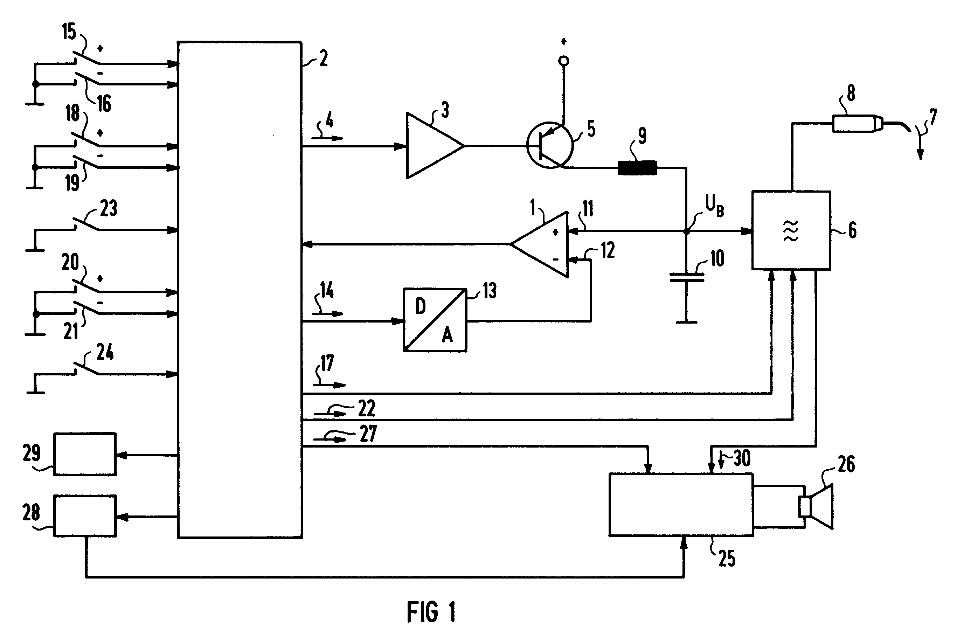

In einem HF-Chirurgiegerät gemäß Figur 1 ist ein Komparator 1 ausgangsseitig mit einem Mikroprozessor 2 zu einer Vergleichsschaltung verbunden, die ein Steuersignal 4 abgibt. Das Steuersignal 4 ist ein binäres Signal mit einer Pulsfrequenz grösser oder gleich 20 kHz und mit einer über den Mikroprozessor 2 einstellbaren Pulsdauer. Das Steuersignal 4 regelt und stabilisiert über eine Steuereinrichtung, bestehend aus einem Treiber 3 und aus einem Leistungstransistor 5, eine gleichgerichtete Betriebsspannung U_{B} eines HF-Generators 6. Der HF-Generator 6, der auch mehrstufig sein kann, erzeugt ein HF-Ausgangssignal 7, das über ein Handstück 8 mit einer Chirurgieelektrode zur Einwirkung auf biologisches Gewebe zur Verfügung steht. Da der Transistor 5 nicht linear, sondern geschaltet betrieben wird, ist eine Siebschaltung, gebildet aus einer Spule 9 und einem Kondensator 10, vorgesehen, um eine konstante Betriebsspannung U_{B} für den HF-Generator, z.B. für dessen Endstufe, zu erzeugen. Die Betriebsspannung U_{B} gelangt auch zum Eingang 11 des Komparators 1. Der Komparator 1 weist einen anderen Eingang 12 auf, dem über einen Digital/Analog-Wandler 13 ein im Mikroprozessor 2 digital erzeugter Sollwert 14 als Analogsignal zugeführt wird. Der Sollwert 14 wird im Mikroprozessor 2 aus einem über die Schalter 15 und 16 vorgebbaren Stellsignal zur Einstellung der HF-Ausgangsleistung und aus einem binären Modulationssignal 17 mit einer Frequenz von z.B. 200 Hz erzeugt, das den HF-Generator 6, z.B. über eine An- und Ausschaltung einer Betriebsspannung, z.B. der gesteuerten Betriebsspannung U_{B}, moduliert.

Eine Erhöhung oder eine Verminderung der vorwählbaren effektiven HF-Ausgangsleistung ist durch eine zeitdauerabhängige Betätigung der Schalter 15 bzw. 16 über den Mikroprozessor 2 gegeben.

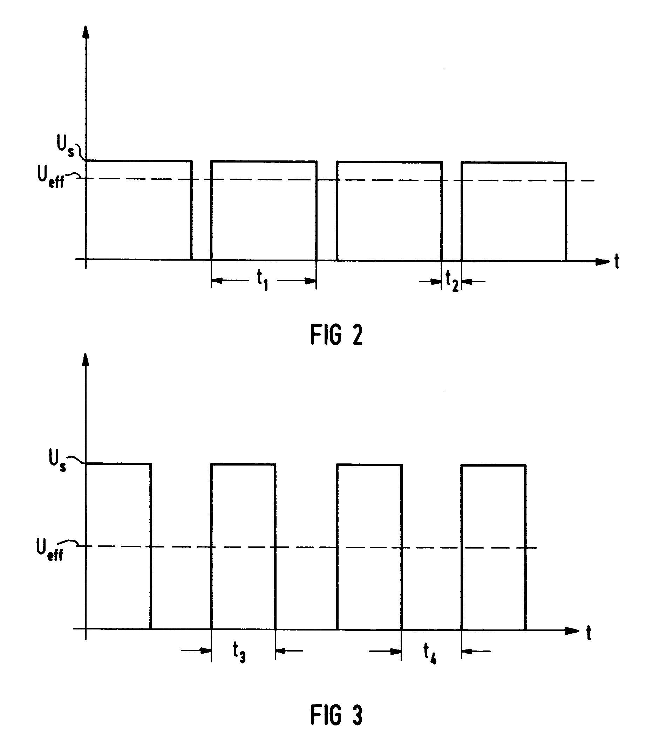

Vorzugsweise am Handstück 8 können Schalter 18 bzw. 19 angeordnet sein, mit denen die Koagulationstiefe über den Mikroprozessor 2 kontinuierlich einstellbar ist. Dazu wird durch eine zeitdauerabhängige Betätigung der Schalter 18 bzw. 19 bei einem z.B. im Mikroprozessor erzeugten Modulationssignal 17, das aus einer Rechteckimpulsfolge bestimmter Taktfrequenz besteht, eine Einstellung der Pulsbreite t₁ bzw. t₃ der Einzelpulse vorgenommen, vergleiche dazu auch die Figuren 2 und 3.

Bauteile zur Einschaltung des Modulationssignals 17 können entfallen, wenn die Pulsbreite t₁ bzw. t₃ so groß wählbar ist, daß unter Fortfall von Pulspausen t₂ bzw. t₄ ein Dauersignal entstehen kann, wodurch der HF-Generator 6 ein konstantes (unmoduliertes) HF-Ausgangssignal abgibt. Mit dem Modulationssignal kann sonach auch der HF-Generator 6 eingeschaltet werden. Das Modulationssignal kann dem Mikrocomputer 2 auch aus einem separaten, nicht dargestellten Generator zugeführt werden, z.B. als eine gleichgerichtete, ungesiebte Wechselspannung.

Aus dem Modulationssignal können im Mikroprozessor 2 weitere, voneinander entkoppelte Signale zur Steuerung von Funktionen im HF-Chirurgiegerät gebildet werden. Dazu kann ein weiteres Signal 22 unabhängig vom Modulationssignal 17 in der Impulsbreite durch zeitabhängiges Betätigen der Schalter 20 bzw. 21 zur Steuerung der Intensität einer Instrumentenbeleuchtung dienen.

Mit einem Schalter 23 kann über den Mikroprozessor 2 der HF-Generator 6 und damit das hochfrequente Ausgangssignal ein- und ausgeschaltet werden. Der Mikroprozessor 2 kann nach Einschaltung eines Schalters 24 auch das Instrumentenlicht steuern, und zwar derart, daß nach Ausschalten des HF-Generators das Instrumentenlicht, z.B. im Handstück 8, noch für eine kurze Zeitspanne, z.B. 7 sec, weiterleuchtet.

Eine Überwachungseinrichtung 25 mit einem angeschlossenen Signalgeber 26 kann von einem aus dem Modulationssignal 17 im Mikroprozessor 2 gebildeten komplementären Signal 27 so gesteuert werden, daß während der Pulspausen t₂ bzw. t₄ im Modulationssignal die Überwachungseinrichtung nicht anspricht, um Fehlalarm zu vermeiden. Des weiteren kann ein derartiges komplementäres Signal 27 auch zur Korrektur einer HF-Leistungsanzeigeeinrichtung 28 im Falle eines modulierten HF-Ausgangssignals herangezogen werden. Eine Verschorfungstiefenanzeige 29 ist ebenfalls sehr günstig ohne besonderen Aufwand an steuernden Bauteilen sowohl aus dem Korrektursignal 27 als auch aus dem Modulationssignal 17 mit Hilfe des Mikroprozessors 2 realisierbar. Wenn der Überwachungseinrichtung 25 ein aus der Betriebsspannung U_{B} abgeleitetes Analogsignal 30 zugeführt wird, kann das Binärsignal 27 auch zur Effektivwertbildung dienen.

Figur 2 zeigt die Hüllkurve eines modulierten HF-Ausgangssignals. Die Pulsbreite (Pulsdauer) t₁ eines jeden Pulses des Modulationssignals 17 ist deutlich größer als die kurze Pause mit der Zeitdauer t₂ zwischen zwei Pulsen. Daraus resultiert eine verhältnismäßig geringe Differenz zwischen der Spitzenspannung Uₛ und der Effektivspannung U_{eff} des Hüllkurvensignals. Daher wird eine nur geringe Koagulationstiefe erreicht.

Gemäß Figur 3 ist das Verhältnis zwischen Pulsdauer t₃ und der Pausendauer t₄ gleich groß gewählt, wodurch eine günstige Koagulationswirkung erreichbar ist. Es ist deutlich dargestellt, daß bei gleicher Effektivspannung U_{eff} in den Figuren 3 und 2 das Verhältnis zur jeweiligen Spitzenspannung Uₛ verschieden und in der Figur 3 erheblich größer ist. Mit dem zur Hüllkurve gemäß Figur 3 gehörenden HF-Ausgangssignal wird somit eine tiefere Koagulation erreicht, ohne daß der einmal gewählte Effektivwert der Ausgangsleistung aufgrund der Modulation nachgestellt werden muß.

Die Pulsfolgefrequenz des Modulationssignals 17 ist bei beiden Darstellungen in den Figuren 2 und 3 gleich groß gewählt, was sich aus den zeitlich übereinstimmenden Pulsanstiegsflanken ergibt. Im Bedarfsfall kann über den Mikroprozessor 2 auch die Pulsfolgefrequenz des dort erzeugten Modulationssignals 17 variiert werden.

Das erfindungsgemäß vorgeschlagene HF-Chirurgiegerät ist mit besonderem Vorteil in der Zahnmedizin einsetzbar, weil dort u.a. die Forderung besteht, die Verschorfungstiefe schnell und problemlos an biologisches Gewebe anpassen zu müssen, um Blutungen im Mundbereich während der Behandlung zu vermeiden.

## Patentansprüche

1. HF-Chirurgiegerät mit einer Vergleichsschaltung (1, 2), die einen Mikroprozessor (2) einschließt und die ein Steuersignal (4) abgibt, das auf die Amplitude einer gleichgerichteten Betriebsspannung (U_{B}) einwirkt, die einen HF-Generator (6) versorgt, dessen Ausgangssignal mit einem Modulationssignal (17) modulierbar ist, wobei der Mikroprozessor (2) aus dem Modulationssignal (17) und aus einem vorgegebenen Stellsignal zur Einstellung einer HF-Ausgangsleistung einen Sollwert (14) bildet, der mit der Betriebsspannung (U_{B}) in der Vergleichsschaltung (1, 2) zur Bildung des Steuersignals (4) verglichen wird, wobei zu jedem Modulationsgrad der dazu notwendige Sollwert (4) ermittelt wird, derart, daß die eingestellte HF-Ausgangsleistung konstant gehalten wird.

2. HF-Chirurgiegerät nach Anspruch 1, bei dem der Mikroprozessor (2) das Modulationssignal (17) als eine Impulsfolge erzeugt, deren Pulse das Ausgangssignal des HF-Generators (6) durch An- und Ausschalten modulieren.

3. HF-Chirurgiegerät nach Anspruch 2, bei dem der Mikroprozessor (2) das Modulationssignal (17) als eine Impulsfolge erzeugt, deren Einzelpulse zur Änderung der Koagulationstiefe in der Pulsbreite (t₁, t₃) einstellbar sind.

4. HF-Chirurgiegerät nach Anspruch 3, mit einem Handstück (8), das Mittel (15, 16; 18, 19) zur Steuerung von Funktionen im HF-Chirurgiegerät aufweist, bei dem wenigstens eines dieser Mittel (18, 19) über den Mikroprozessor (2) auf die Pulsbreite (t₁, t₃) des Modulationssignals (17) derart einwirkt, daß mit zunehmender Pulsbreite (t₁, t₃) unter Fortfall von Pulspausen (t₂, t₄) ein Dauersignal entstehen kann.

5. HF-Chirurgiegerät nach Anspruch 2, bei dem der Mikroprozessor (2) das Modulationssignal (17) als eine Impulsfolge erzeugt, aus der zwei voneinander entkoppelte Signale (17, 22) gebildet werden, deren Einzelpulse unabhängig voneinander in der Pulsbreite (t₁, t₃) einstellbar sind.

6. HF-Chirurgiegerät nach Anspruch 5, bei dem das eine entkoppelte Signal das Modulationssignal (17) bildet, aus dem ein Komplementärsignal (27) zur Steuerung einer Überwachungseinrichtung (25) gebildet wird und bei dem das andere entkoppelte Signal (22) zur Instrumentenbeleuchtung dient.

7. HF-Chirurgiegerät nach einem der Ansprüche 1 bis 7, bei dem die Vergleichsschaltung aus dem Mikroprozessor (2) und einem damit ausgangsseitig verbundenen Komparator (1) gebildet ist, aus dessen Ausgangssignal im Mikroprozessor (2) das Steuersignal (4) erzeugt wird, das über eine Steuereinrichtung (3, 5) auf die gleichgerichtete Betriebsspannung (U_{B}) einwirkt.

8. HF-Chirurgiegerät nach einem der Ansprüche 1 bis 7, bei dem der Sollwert (14) im Mikroprozessor (2) digital erzeugt und über einen Digital/Analog-Wandler (13) als Analogsignal dem einen Eingang (12) der Vergleichsschaltung (1, 2) zugeführt wird, deren anderer Eingang (11) mit der gesteuerten Betriebsspannung (U_{B}) verbunden ist.

## Claims

1. HF surgery device with a differential connection (1, 2) which includes a microprocessor (2) and which emits a control signal (4) which acts on the amplitude of a rectified operating voltage (U_{B}), which supplies a HF generator (6), the output signal of which can be modulated with a modulation signal (17), whereby the microprocessor (2) forms from the modulation signal (17) and from a specified actuating signal for the adjustment of a HF output a desired value (14) which is compared with the operating voltage (U_{B}) in the differential connection (1, 2) for the formation of the control signal (4), whereby for each modulation depth the desired value (4) necessary for this is determined in such a way that the HF output set is kept constant.

2. HF surgery device according to claim 1, where the microprocessor (2) generates the modulation signal (17) as a pulse train, the pulses of which modulate the output signal of the HF generator (6) through connection and disconnection.

3. HF surgery device according to claim 2, where the microprocessor (2) generates the modulation signal (17) as a pulse train, the individual pulses of which can be adjusted to change the coagulation depth in the pulse width (t₁, t₃).

4. HF surgery device according to claim 3, having a handpiece (8) which has means (15, 16; 18, 19) to control functions in the HF surgery device, where at least one set of these means (18, 19) acts by way of the microprocessor (2) on the pulse width (t₁, t₃) of the modulation signal (17) in such a way that with increasing pulse width (t₁, t₃) with the omission of pulse pauses (t₂, t₄) a continuous signal can arise.

5. HF surgery device according to claim 2, where the microprocessor (2) generates the modulation signal (17) as a pulse train, from which two signals (17, 22) decoupled from one another are formed, the individual pulses of which can be adjusted independently of one another in the pulse width (t₁, t₃).

6. HF surgery device according to claim 5, where the one decoupled signal forms the modulation signal (17), from which a complementary signal (27) is formed to control a monitoring device (25) and where the other decoupled signal (22) serves to illuminate the instruments.

7. HF surgery device according to one of claims 1 to 7, where the differential connection is formed from the microprocessor (2) and a comparator (1) connected thereto on the output side, from the output signal of which in the microprocessor (2) the control signal (4) is generated, which acts by way of a control device (3, 5) on the rectified operating voltage (U_{B}).

8. HF surgery device according to one of claims 1 to 7, where the desired value (14) in the microprocessor (2) is digitally generated and is supplied by way of a digital-to-analog converter (13) as analog signal to the one input (12) of the differential connection (1, 2), the other input (11) of which is connected to the controlled operating voltage (U_{B}).

## Revendications

1. Appareil chirurgical HF comportant un circuit comparateur (1,2), qui comporte un microprocesseur (2) et délivre un signal de commande (4), qui agit sur l'amplitude d'une tension de service redressée (U_{B}), qui alimente un générateur HF (6), dont le signal de sortie peut être modulé par un signal de modulation (17), le microprocesseur (2) formant, à partir du signal de modulation (17) et d'un signal de réglage prédéterminé, pour le réglage d'une puissance de sortie HF, une valeur de consigne (14), qui est comparée à la tension de service (U_{B}) dans le circuit comparateur (1,2) pour la formation du signal de commande (4), la valeur de consigne nécessaire (4) étant déterminée pour chaque degré de modulation de telle sorte que la puissance de sortie HF réglée est maintenue constante.

2. Appareil chirurgical HF suivant la revendication 1, dans lequel le microprocesseur (2) produit le signal de modulation (17) sous la forme d'une suite d'impulsions, dont les impulsions modulent le signal de sortie du générateur HF (6), par application et suppression.

3. Appareil chirurgical HF suivant la revendication 2, dans lequel le microprocesseur (2) produit le signal de modulation (17) sous la forme d'une suite d'impulsions, dont dont les durées (t₁, t₃) des impulsions individuelles sont réglables pour modifier l'intensité de coagulation.

4. Appareil chirurgical HF suivant la revendication 3, comportant une pièce à main (8), qui comporte des moyens (15,16;18,19) pour commander des fonctions dans l'appareil chirurgical HF, au moins l'un de ces moyens (18,19) agissant par l'intermédiaire du microprocesseur (2) sur la durée (t₁, t₃) des impulsions du signal de modulation (17) de telle sorte que lorsque la durée (t₁, t₃) d'impulsions augmente, un signal continu peut apparaître, moyennant la suppression de pauses (t₂, t₄) entre impulsions.

5. Appareil chirurgical HF suivant la revendication 2, dans lequel le microprocesseur (2) produit le signal de modulation (17) sous la forme d'une suite d'impulsions, à partir de laquelle sont formés deux signaux (17,22) découplés l'un par rapport à l'autre et dont les durées (t₁, t₃) des impulsions individuelles peuvent être réglées indépendamment l'une de l'autre.

6. Appareil chirurgical HF suivant la revendication 5, dans lequel un signal découplé forme le signal de modulation (17), à partir duquel un signal complémentaire (27) est formé pour la commande d'un dispositif de contrôle (25), et dans lequel l'autre signal découplé (22) sert à éclairer l'instrument.

7. Appareil chirurgical HF suivant l'une des revendications 1 à 7, dans lequel le circuit comparateur est formé par le microprocesseur (2) et par un comparateur (1) relié, côté sortie, à ce microprocesseur et à partir du signal de sortie duquel est produit, dans le microprocesseur (2), le signal de commande (4), qui agit, par l'intermédiaire d'un dispositif de commande (3,5), sur la tension de service redressée (U_{B}).

8. Appareil chirurgical HF suivant l'une des revendications 1 à 7, dans lequel la valeur de consigne (14) est produite numériquement dans le microprocesseur (2) et est envoyée, par l'intermédiaire d'un convertisseur numérique/analogique (13), en tant que signal analogique, à une entrée (12) du circuit comparateur (1,2), dont l'autre entrée (11) est reliée à la tension de service commandée (U_{B}).
